# EUROPÄISCHE PATENTANMELDUNG

(11) **EP 1 764 087 A2**
(43) Veröffentlichungstag der Anmeldung: **21.03.2007**
(21) Anmeldenummer: 06120664.5
(22) Anmeldetag: 14.09.2006
(51) Int. Cl.: A61K 8/97, A61Q 1/02

(54) **Kosmetische Zubereitungen zur dekorativen Anwendung auf der Haut**

(30) Priorität: 16.09.2005 DE 102005045147
(71) Anmelder: Beiersdorf AG, 20253 Hamburg (DE)
(72) Erfinder: Schäfer, Jessica, 22299, Hamburg (DE); Warnke, Katja, Hamburg, 20257 (DE); Fiedler, Dorothe, 22335, Hamburg (DE)

(57) **Zusammenfassung**

Kosmetische Zubereitung zur dekorativen Anwendung auf der Haut, welche ein oder mehrere Farbpigmente enthält und dadurch gekennzeichnet ist, daß sie ferner ein oder mehrere Ligninsulfonate enthält.

## Beschreibung

Die Erfindung betrifft kosmetische Zubereitungen zur dekorativen Anwendung auf der Haut, welche ein oder mehrere Ligninsulfonate und Farbpimente enthalten.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Dekorative Kosmetika - wie beispielsweise Gesichts-Make-up-Formulierungen - sollen der (Gesichts-) Haut ein natürliches Aussehen verleihen, einen blassen Teint optisch auffrischen sowie farbliche Unregelmäßigkeiten der Haut ausgleichen. Diese Effekte sollen nach Möglichkeit den ganzen Tag über halten, insbesondere ohne dass die Zubereitung auf die getragene Kleidung abfärbt.

Zunehmend wird aber neben diesen rein dekorativen Anforderungen, insbesondere von sogenannten "Foundations", auch eine Schutzfunktion gewünscht, z. B. vor schädigenden Umwelteinflüssen, insbesondere vor UV-Strahlung.
Als Foundation bezeichnet man dabei flüssige oder pastöse Make-up-Präparate auf Emulsionsbasis, die meist hautfarben sind und auf das Gesicht oder auch auf die Wangen aufgetragen werden. Sie verleihen diesen ein gleichmäßiges, gesundes, gebräuntes oder rötliches Aussehen.

Üblicherweise werden in dekorativen Kosmetika Farbpigmente eingesetzt, um diesen Anforderungen gerecht zu werden, da diese sowohl rein dekorative als auch zusätzliche Schutzfunktionen erfüllen können. Allerdings führt der Einsatz solcher Pigmente beim Auftrag der Produkte oft zu einem unerwünschten, maskenhaften Effekt, der insbesondere durch die mangelnde Transparenz der Produkte noch verstärkt wird, da Farbpigmente die Haut mehr oder weniger vollständig abdecken, dabei den natürlichen Hautton aber nicht vollkommen nachstellen können.

Reduziert man den Gehalt der Farbpigmente, so geht zwar der maskenhafte Effekt zurück, allerdings läßt sich auch der gewünschte Farbeffekt üblicherweise nicht mehr erreichen und Unregelmäßigkeiten der Haut werden nicht mehr zuverlässig ausgeglichen. Auch der Einsatz von wasser- oder öllöslichen Farbstoffen hilft hier nicht weiter, da diese dazu neigen, die Haut einzufärben, so dass auch nach der Entfernung des Make-ups noch Rückstände auf der Haut sichtbar bleiben.

Aufgabe der vorliegenden Erfindung war es daher, auf einfache und preiswerte Weise zu kosmetische Zubereitungen zur dekorativen Anwendung auf der Haut zu gelangen, welche die genannten Nachteile nicht aufweisen.

Überraschend und für den Fachmann nicht vorauszusehen war, daß eine kosmetische Zubereitung zur dekorativen Anwendung auf der Haut, welche ein oder mehrere Farbpigmente enthält und dadurch gekennzeichnet ist, daß sie ferner ein oder mehrere Ligninsulfonate enthält,
den Nachteilen des Standes der Technik abhelfen würden.

Die Zubereitungen gemäß der vorliegenden Erfindung stellen in jeglicher Hinsicht überaus befriedigende Präparate dar, welche sehr gute sensorische und kosmetische Eigenschaften - wie beispielsweise die Verteilbarkeit auf der Haut - zeigen. Überraschenderweise wurde gefunden, dass Produkte der dekorativen Kosmetik - insbesondere sogenannte Foundations - welche ein oder mehrere Ligninsulfonate enthalten, bei ihrer Anwendung einen wünschenswerten, sehr natürlichen Braunton auf der Haut erzielen und keinen unerwünschten, maskenhaften Effekt hervorrufen. Dabei zeichnen sich die Zubereitungen im Sinne der vorliegenden Erfindung nicht nur durch eine sehr natürliche Färbung der Haut aus, sondern sie gleichen zusätzlich vorhandene Unregelmäßigkeiten optisch aus. Desweiteren haben die erfindungsgemäßen Zubereitungen - auch ohne den additiven Einsatz von Lichtschutzfiltern - eine zusätzliche Schutzfunktion, die sich in einem erhöhten LSF, UVA Balance sowie PPD (persistent pigment darkening) Wert widerspiegelt.

Ferner bilden Produkte im Sinne der vorliegenden Erfindung einen angenehm weichen, dabei aber übertragungsresistenten ("transferresistance") Film auf der Haut, der einem Abfärben des Produkts auf die Kleidung entgegenwirkt, trotzdem leicht und rückstandslos entfernbar ist und hierbei die Haltbarkeit des Produktes auf der Haut entscheidend verbessert.

Ligninsulfonsäure ist das beim Sulfit-Aufschluß von Holz zur Gewinnung von Cellulose anfallende Reaktionsprodukt aus nativem Lignin und schwefliger Säure. Bei diesem Aufschlußverfahren wird Lignin - ein hochmolekularer, aromatischer Stoff, der in verholzenden Pflanzen die Räume zwischen den Zellmembranen ausfüllt und diese hierdurch versteift und zu Holz werden läßt - an den C₃-Seitenketten der Phenylpropan-Grundeinheiten sulfoniert.

In Abhängigkeit von den beim Aufschlußverfahren verwendeten Basen resultieren wasserlösliche Natrium-, Ammonium-, Calcium- oder Magnesium-Salze der Ligninsulfonsäure. Angaben zum M_{R} der Ligninsulfonsäure. variieren mit Werten von ca. 10.000 bis 200.000; die Anzahl der Sulfonsäure-Gruppen beträgt ca. 2 pro 5 bis 8 Phenylpropan-Einheiten. Ligninsulfonsäure und ihre Salze, die Ligninsulfonate, sind Hauptbestandteil der SulfitAblaugen, aus denen sie als braune Pulver isoliert werden können. Die isolierten Produkte finden breite technische Verwendung, z. B. als Dispergatoren in Zement- und Gipsmörteln, als Bohrspülflüssigkeiten sowie als Flotationshilfsmittel, als Zusatz bei der Futtermittelpalettierung sowie zur Formsandbindung in Gießereien.

Vorteilhafte Ligninsulfonate im Sinne der vorliegenden Erfindung sind Calciumligninsulfonat (CAS-Nr. :8061-52-7) und Natriumligninsulfonat (CAS-Nr.: 8061-51-6), insbesondere die unter den Handelbezeichnungen Ultrazine NA und Vanisperse CB bei der Firma Borregaard Lignotech erhältlichen Ligninsulfonate.

Besonders vorteilhafte Zubereitungen im Sinne der vorliegenden Erfindung enthalten 0,01 bis 20 Gew.-%, vorteilhaft 0,5 bis 15 Gew.-%, besonders 1 bis 7 Gew.-% an einem oder mehreren Ligninsulfonaten (jeweils bezogen auf das Gesamtgewicht der Zubereitung).

Es ist vorteilhaft im Sinne der vorliegenden Erfindung, den Gehalt an einem oder mehreren Farbpigmenten wie 0,01 bis 20, vorteilhaft 0,5 bis 15, besonders von 1 bis 10 Gew.-% zu wählen (jeweils bezogen auf das Gesamtgewicht der Zubereitung).

Dabei zeichnen sich die Zubereitungen im Sinne der vorliegenden Erfindung insbesondere dadurch aus, dass sie die Haut sehr natürlich aussehen lassen und gleichzeitig vorhandene Unregelmäßigkeiten optisch ausgleichen, wobei für die gleiche Deckkraft üblicherweise weniger Farbpigmente benötigt werden als bei den Zubereitungen des Standes der Technik.

Die Auswahl der Farbpigmente erfolgt in erster Linie mit dem Ziel, einen modisch aktuellen Farbton zu erzielen, jedoch im Rahmen der natürlichen Hautfärbung zu bleiben. Dabei muß allerdings beachtet werden, daß alle Farbstoffe und Pigmente hinsichtlich ihrer Verwendung gesetzlichen Bestimmungen unterliegen, in Deutschland beispielsweise der Verordnung über kosmetische Mittel. Dementsprechend können die Farbpigmente aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid und/oder Eisenoxide (z. B. Fe₂O₃, Fe₃O₄, FeO(OH)).

Vorteilhaft im Sinne der vorliegenden Erfindung sind ferner Formulierungen mit einem Gehalt an Effektpigmenten (Perlglanz- und/oder Interferenzpigmenten), welche in den erfindungsgemäßen Formulierungen überraschenderweise besonders gut zur Geltung kommen. Bevorzugt sind insbesondere die im folgenden aufgelisteten Arten von Perlglanzpigmenten:
Natürliche Perlglanzpigmente, wie z. B.
   - "Fischsilber" (Guanin/Hypoxanthin-Mischkristalle aus Fischschuppen) und
   - "Perlmutt" (vermahlene Muschelschalen)
Monokristalline Perlglanzpigmente wie z. B. Bismuthoxychlorid (BiOCl)
Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Erfindungsgemäße Zubereitungen, die neben der Pigmentphase Öl- und Wasserphasen enthalten, sind beispielsweise Emulsionen oder Hydrodispersionen. Emulsionen im Sinne der Erfindung können O/W-, W/O-, W/Si- (Wasser-in-Silikonöl) oder Feststoff-Emulsionen sein. Diese Emulsionen können auch fest, sprühbar oder fließfähig sein. Darüber hinaus können solche Systeme zusätzlich in Öl- oder Wasserphasen dispergierte Gasblasen enthalten.

Die erfindungsgemäßen Zubereitungen können dementsprechend in Form von Stiften, Schäumen (so genannten Mousse), Feststoff-Emulsionen (d. h. Emulsionen, welche durch Feststoffe stabilisiert sind wie z. B. Pickering-Emulsionen), sprühbaren Emulsionen oder Hydrodispersionen vorliegen.

Erfindungsgemäße Emulsionen enthalten neben einer Ölphase, die aus Ölen, Fetten, Wachsen oder deren Mischungen bestehen kann, Wasser und vor allem Emulgatoren sowie gegebenenfalls weitere Hilfsstoffe wie Coemulgatoren, Konsistenzgeber, Verdicker, Stabilisatoren, Polyole, Hydrotrope, Konservierungsmittel, Wirkstoffe, UV-Filter sowie Puder- und Füllstoffe.

So enthalten erfindungsgemäße O/W-Emulsionen mindestens einen Emulgator mit einem HLB Wert > 7 und gegebenenfalls einen Coemulgator. Diese Emulgatoren können vorteilhaft gewählt werden aus der Gruppe der nichtionischen oder anionischen Emulgatoren.

Unter den nichtionischen Emulgatoren befinden sich vorzugsweise
a) Partialfettsäureester und Fettsäureester mehrwertiger Alkohole und deren ethoxilierte Derivate (z. B. Glycerylmonostearate, Sorbitanstearate, Glyceryl Stearyl Citrate, Sucrose Stearate)
b) ethoxilierte Fettalkohole und Fettsäuren.

O/W-Emulgatoren, mit denen sich erfindungsgemäße O/W-Emulsionen herstellen lassen, sind vor allem nichtionische oder anionische Emulgatoren. Vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemäßen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemäßen Zubereitungen durch den Einsatz weiterer Emulgatoren erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusöl, Polyglyceryl-4-isostearat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

Den erfindungsgemäßen O/W-Emulsionen können hydrophile Gelbildner beispielsweise zur Stabilisierung zugesetzt werden. Vorteilhafte Gelbildner für derartige Zubereitungen sind z. B.

Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind Pemulen® TR 1, Pemulen® TR 2 und Pemulen® TRZ von der Fa. Noveon.

Auch Carbomere sind vorteilhafte Gelbildner für derartige Zubereitungen. Carbomere sind Polymere der Acrylsäure, insbesondere Acrylat-Alkylacrylat-Copolymere. Vorteilhafte Carbomere sind beispielsweise Carbopol 980, Carbopol 981, Carbopol 1342, Carbopol 1382, Carbopol 2984 und Carbopol 5984 sowie Carbopol ETD 2020, Carbopol ETD 2050 und Carbopol Ultrez 10 der Fa. Noveon. Weitere vorteilhafte Gelbildner für derartige Zubereitungen sind Xanthan Gummi, Cellulose Derivate oder Johannisbrotkernmehl sowie Acrylamidmethylpropylsulfonsäurepolymere (so genannte AMPS-Polymere) insbesondere hydrophobisierte Acrylamidomethylpropylsulfonsäure-Polymere, die am AMPS-Grundgerüst über PEG-Ketten (mit 2 bis 200, vorteilhaft 5 bis 25 Ethoxyeinheiten) gebundene Alkylketten tragen, wobei die Kettenlänge im Bereich zwischen C₆ und C₂₂ liegt.

Zur Verbesserung des Hautgefühls und der Stabilität sowie zur Einstellung der Viskosität können beispielsweise Verdicker und Konsistenzgeber zugesetzt werden. Besonders vorteilhafte Konsistenzgeber sind hierbei Glycerylstearat, -palmitat oder -oleat sowie Fettalkohole, vor allem unverzweigte gesättigte mit 10 bis 24 C-Atomen. Auch Wachse können als Konsistenzgeber eingesetzt werden.

Weiterhin vorteilhaft können Filmbildner enthalten sein, welche beispielsweise eine gleichmäßigere Verteilung der Pigmente auf der Haut und eine Verbesserung der Transferresistenz der auf der Haut aufgetragenen Zubereitung bewirken. Besonders vorteilhafte Filmbildner sind hierbei VP Eicosene Copolymer, VP Hexadecene Copolymer, Sucrose Acetate Isobutyrate sowie Trimethylsiloxysilicate.

Eine vorteilhafte erfindungsgemäße O/W-Emulsion ist beispielsweise eine O/W-Maskara. Es handelt sich hierbei bevorzugt um ein mit Triethanolamin verseiftes Stearatsystem, zu dem noch weitere Emulgatoren gegeben werden können, mit hohen Mengen an Wachsen, Pigmenten und Filmbildnern. Außer Triethanolamin kann auch Aminomethylpropanol als Verseifungsmittel für die Stearinsäure verwendet werden. Vorzugsweise liegt die Einsatzkonzentration von Triethanolamin-Stearat bei 5 bis 8 Gew.-%, die der Pigmente bei 8 bis 12 Gew.-%, Wachse von 8 bis 15 Gew.-%. Als Filmbildner können sowohl hydrophile als auch lipophile Filmbildner eingesetzt werden. Weiterhin ist der Einsatz von Verdickern wie z. B. Hydroxyethylcellulose sinnvoll.

Die Wachse können aus der Gruppe der Bienenwachse und ihrer Teilfraktionen sowie der Bienenwachsderivate, der Gruppe der paraffinischen Kohlenwasserstoffwachse wie mikrokristalline Wachse und Ozokerite sowie aus der Gruppe der homopolymeren Polyethylene gewählt werden. Ebenfalls vorteilhaft können natürliche Wachse wie Reiswachs, Candellilawachs, Carnaubawachs, Japanwachs oder Schellackwachs eingesetzt werden.

Es kann sinnvoll sein, zur Herstellung von erfindungsgemäßen O/W-Emulsionen zunächst eine Pigmentphase aus allen Feststoffen, Pigmenten und/oder Füllstoffen herzustellen, die ggf. - falls notwendig - in einem oder mehreren Ölen vordispergiert werden kann. Diese Dispersion kann hierbei zusätzlich weitere Bestandteile der Ölphase enthalten, zum Beispiel zur besseren Benetzung einen Emulgator. Die Pigmentphase wird vor der Phasenvereinigung (Wasser - Öl) der Ölphase zugesetzt.
Die erfindungsgemäßen Ligninsulfonate werden ebenfalls vor der Phasenvereinigung der Wasserphase zugesetzt. In Abhängigkeit des gewählten Ligninsulfonat-Typs, kann es nötig sein, die Wasserphase zu Verbesserung der Löslichkeit zu erwärmen.

Weitere erfindungsgemäße Zubereitungen können W/O- bzw. Wasser-in-Silikonöl- (W/S-) Emulsionen sein. Vorteilhaft im Sinn der Erfindung sind dabei W/O- oder W/S-Emulsionen, die
- ein oder mehrere Silikonemulgatoren (W/S) mit einem HLB-Wert ≤ 8 oder ein oder W/O-Emulgatoren mit einem HLB-Wert < 7 oder eine Mischung dieser Emulgatoren und gegebenenfalls
- ein oder mehrere O/W-Emulgatoren mit einem HLB-Wert > 10 enthalten.

Die erfindungsgemäßen W/O-Emulsionen enthalten vorteilhaft mindestens 20 Gew.-% an Ölphase, wobei die Ölphase Silikonöle enthalten oder ganz aus Silikonölen bestehen kann (W/S-Emulsionen). Weiterhin können die erfindungsgemäßen W/O- oder W/S-Emulsionen Feststoffe in einem Bereich von 5 bis 25 Gew.-% enthalten, wobei sich die Feststoffphase bevorzugt aus Pigmenten und Füllstoffen zusammen setzt.

Die Silikonemulgatoren können vorteilhaft aus der Gruppe enthaltend Alkyldimethiconcopolyole wie z. B. Cetyl PEG/PPG 10/1 Dimethiconcopolyol (ABIL® EM 90 der Fa. Goldschmidt AG) oder Lauryl PEG/PPG- 18/18 Dimethicone (Dow Corning® 5200 Formulation Aid der Fa. Dow Corning Ltd.) und Dimethiconecopolyole wie z. B. PEG-10 Dimethicone (KF-6017 der Fa. Shin Etsu), PEG/PPG- 18/18 Dimethicone (Dow Corning Formulation aid 5225C der Fa. Dow Corning Ltd.) oder PEG/PPG-19/19 Dimethicone (Dow Corning BY-11 030 der Fa. Dow Corning Ltd.) gewählt werden.

Die W/O-Emulgatoren mit einem HLB-Wert < 7 können vorteilhaft aus der Gruppe enthaltend Sorbitanstearat, Sorbitanoleat, Glycerylisostearat, Polyglyceryl-3-oleat, Pentaerythrithylisostearat, Methylglucosedioleat, PEG-7-hydriertes Ricinusöl, Polyglyceryl-4-isostearat, Hexyllaurat, Sorbitanisostearat, Polyglyceryl-2-dipolyhydroxystearat, Polyglyceryl-3-Diisostearat, PEG-30-dipolyhydroxystearat, Diisostearoylpolyglyceryl-3-düsostearat, Polyglyceryl-3-dipolyhydroxystearat, Polyglyceryl-4-dipolyhydroxystearat, Polyglyceryl-3-dioleat und Wollwachsalkohol (Eucerit) gewählt werden.

Die O/W-Emulgatoren mit einem HLB-Wert > 10 können vorteilhaft aus der Gruppe enthaltend Lecithin, Trilaureth-4-phosphat, Polysorbate-20, Polysorbate-60, PEG-22-Dodecylglycol Copolymer, Sucrosestearat und Sucroselaurat gewählt werden.

Zur Stabilisierung der erfindungsgemäßen Emulsionen gegen Sedimentierung oder Flockung der Wassertröpfchen kann es von Vorteil sein, darüber hinaus noch Ölverdicker hinzuzusetzen. Besonders geeignet sind hierfür Aerosil (ggf. modifizierte pyrogene Kieselsäure), Silikate wie Bentonit, Hectorit oder Montmorillonit (zur Optimierung der Eigenschaften ggf. oberflächenbehandelt) sowie Magnesium- oder Aluminiumstearat.

Weiterhin vorteilhaft kann der Einsatz von Wachsen sein, insbesondere von silikonmodifizierten Wachsen wie z. B. Cetearyl Methicone, Siliconyl Beeswax oder C30-45 Alkyl Dimethicone.

Ebenso können der Ölphase der erfindungsgemäßen Emulsionen weitere Silikonöle oder silikonmodifizierte Öle zugesetzt werden. Als vorteilhafte Silikonöle können Dimethicone, Phenyl Trimethicone oder Bis-Phenylpropyl Dimethicone eingesetzt werden. Vorteilhafte silikonmodifizierte Öle sind z. B. Methyl Ester Siloxane (GE Bayer SF 1318 der Fa. Bayer) oder Phenylpropyl Dimethylsiloxysilicat (GE Bayer Silshine 151 der Fa. Bayer).

Die erfindungsgemäße Emulsion kann zusätzlich Filmbildner enthalten, um z. B. eine gleichmäßigere Verteilung der Pigmente auf der Haut zu erhalten und die Transferresistenz der auf die Haut aufgetragenen Zubereitung zu verbessern.

Vorteilhafte Filmbildner sind z. B. Trimethylsiloxysilicate (z. B. SR 1000 GE der Fa. Bayer), Silikon Acrylate Copolymere (z. B. TIB4-200 der Fa. Dow Corning oder KP- 561 der Fa. Shin Etsu) oder Trimethyl Pentaphenyl Trisiloxane (Dow Corning 555 Cosmetic Fluid der Fa. Dow Corning Ltd.).

Darüber hinaus kann es von Vorteil sein, der Rezeptur nicht-partikuläre sensorische Additive zuzusetzen, welche aus der Gruppe der Dimethiconole (z. B. Dow Corning 1503 Fluid der Fa. Dow Corning Ltd.), der Siliconcopolymere (z. B. Divinyldimethicone/Dimethicone Copolymer, Dow Corning HMW 2220 der Fa. Dow Corning Ltd.) oder der Siliconelastomere (z. B. Dimethicone Crosspolymer, Dow Corning 9040 Silicone Elastomer Blend der Fa. Dow Corning Ltd.) gewählt werden können.

Zur Herstellung von erfindungsgemäßen W/O- und W/Si-Emulsionen wird zunächst eine Pigmentphase aus allen Feststoffen, Pigmenten und/oder Füllstoffen hergestellt, die ggf. - falls notwendig - in Wasser sowie weiteren Bestandteilen der Wasserphase, wie z. B. Glycerin dispergiert werden. Im Falle der W/Si Emulsion werden die Pigmente vorzugsweise in einem oder mehreren Ölen dispergiert. Diese Dispersion kann hierbei zusätzlich weitere Bestandteile der Ölphase enthalten. Die Pigmentphase wird vor der Phasenvereinigung (Wasser - Öl) der Ölphase zugesetzt.

Die Ligninsulfonate werden in beiden Fällen der Wasserphase vor der Phasenvereinigung zugesetzt und ggf. unter Erwärmung gelöst. Im Falle der W/O Emulsion sollte der Zusatz der Ligninsulfonate vor der Pigmentdispergierung erfolgen, da die Ligninsulfonate eine Verbesserung der Dispergierbarkeit herbeiführen.

Alle beschriebenen Zubereitungen sind mit den verschiedensten Ölen erhältlich. Wie dem Fachmann bekannt, sind jedoch spezifische Öle, in Abhängigkeit der jeweiligen Produktform, bevorzugt einzusetzen. Auch eine Mischungen der Öle ist denkbar und für verschiedene gewünschte Eigenschaften der Zubereitung unumgänglich. Auch hier sind dem Fachmann die bevorzugten Mischungen und Konzentrationsbereiche der einzelnen Öle bekannt.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlän- ge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 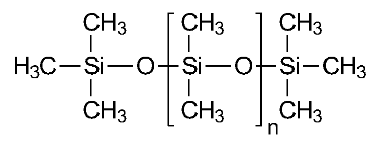 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCl: Amodimethicone) und Silikonwachse, z. B. Poly- siloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Weitere Hilfsstoffe, die sowohl in W/O- als auch in O/W-Emulsionen eingesetzt werden können, sind beispielsweise Konservierungsmittel, Puder- und Füllstoffe, Hydrotrope und Wirkstoffe.

Geeignete Konservierungsmittel für Emulsionen sind Parabene, Phenoxyethanol, lodopropinylbutylcarbamat, Sorbinsäure, DMDM Hydantoin, Diazolidinylharnstoff, Sorbinsäure (vor allem bei pH-Werten kleiner 5) und Benzylalkohol. Es ist bekannt, dass Mischungen von Konservierungsmitteln synergistische Effekte zeigen, weshalb i. d. R. verschiedene Kombinationen mikrobiologischen Belastungstests unterzogen werden. Es kann darüber hinaus vorteilhaft sein, die Konservierungsmittel in anderen Stoffen gelöst einzusetzen, vor allem Polyole finden hier vorteilhaft Verwendung.

Geeignete Puder- und Füllstoffe sind insbesondere Stärke und Stärkederivate (z. B. Aluminum- oder Natrium-Stärke-Octenylsuccinat, Distärkephosphat), Talkum, Glimmer (Mica), Nylon (z. B. Nylon-12 oder Nylon-6/12), pyrogene Kieselsäuren, Kaolin oder partikuläres Polyethylen zum Einsatz. Die Teilchengröße der Rohstoffe liegt bevorzugt zwischen 1 und 30 µm und ihr Brechungsindex ist vorteilhaft zwischen 1 und 5.

Geeignete Hydrotrope sind aufgrund ihrer guten Hautpflegeeigenschaften insbesondere wasserlösliche organische Verbindungen wie z. B. Ethanol, Isopropanol, Harnstoff, Ethylhexyloxyglycerin, Methylpropandiol, Glycerin oder Alkandiole mit 2 bis 10 Kohlenstoffatomen wie Propylenglykol, Butylenglykol usw.

Es sind zahlreiche Wirkstoffe bekannt, die auch in der vorliegenden Erfindung vorteilhaft zum Einsatz kommen können. Besonders geeignet sind Vitamine (A, B, C, E und ihre Derivate und Provitamine), Antioxidantien (Flavonoide, Isoflavonoide und ihre Derivate). Auch Komplexbildner wie EDTA und Iminodibernsteinsäure können vorteilhaft im Sinne der vorliegenden Erfindung eingesetzt werden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele: Emulsionen

| **Inhaltsstoff (INCI-Bezeichnung)** | **Anteil in Gew.-%** | | | | |
|---|---|---|---|---|---|
| | **Fdt.I** | **Fdt.II** | **Fdt. III** | **Fdt. IV** | **Getönte Tagescreme** |
| Stearic Acid | | | 1,50 | 2,00 | |
| Sodium Stearate | 2,00 | | | | |
| Glycerinmonostearat | | | 2,0 | 1,50 | |
| PEG-100 Stearate | | | 1,20 | 0,60 | |
| Glyceryl Stearat | 1,00 | | | | |
| Glyceryl Stearat Citrat | | | | | 3,50 |
| Octyldodecanol | | | 7,00 | | 3,00 |
| Polyglyceryl-4 Isostearate | | 2,00 | | | |
| Cetyl PEG/PPG-10/1 Dimethicone | | 1,00 | | | |
| Cyclomethicone | | 25 | 6,00 | 12,00 | 3,00 |
| Dimethicone | 5,00 | | | 1,00 | |
| Ceatearyl Alcohol | 2,00 | | | | 1,50 |
| Cera Microcristallina | 0,50 | | | | |
| Paraffinum Liquidum | 1,50 | | | | |
| Squalane | | | | | 2,00 |
| Isoeicosane | | 5,00 | | | |
| Diisostearyl Malate | | | 2,00 | | |
| Caprylic/Capric Triglyceride | 4,00 | | | | |
| Dicaprylyl Carbonate | 1,50 | | | 0,50 | |
| Dimethicone / Vinyl Dimethicone Copolymer (z. B. Dow Corning HMW 2220) | | | 1,00 | | |
| Magnesium Sulfate | | 1,00 | | | |
| Pentaerythrithyl Tetracocoate | | | 2,00 | | |
| Shea Butter | | | | | 5,00 |
| Carbomer | 0,07 | | | | 0,60 |
| Xanthan Gum | | | 0,30 | 0,20 | |
| Magnesium Aluminium Silikate (z. B. Veegum) | | | 1,00 | 0,80 | |
| Disteardimonium Hectorite | | 1,00 | | | |
| Propylene Carbonate | | 0,20 | | | |
| Glycerin | 5,50 | 3,00 | 5,00 | 3,00 | 10,00 |
| Chinacridon | | | 1,50 | 0,60 | 0,30 |
| Glimmer | | | 1,00 | | |
| Interferenzpigmente | | | | | 3,00 |
| Eisenoxide | | | 3,00 | 3,60 | |
| Titandioxid | | | 2,50 | 5,00 | |
| Talkum | | | 5,00 | 2,00 | |
| Nylon-12 | | 5,00 | | | |
| Octyl Methoxy Cinnamat | | | | | 2,00 |
| mikronisiertes Titandioxid | | | | | 2,00 |
| Butyl Methoxy Dibenzoylmethan | | | | | 0,50 |
| Tocopherolacetat | | | 1,00 | | 1,00 |
| Ligninsulfonat | 5,50 | 3,00 | 5,00 | 2,50 | 1,50 |
| Antioxidantien, Konservierungsmittel, Neutralisationsmittel, Parfum etc. | q. s. | q. s. | q. s. | q. s. | q. s. |
| Wasser | ad 100 | ad 100 | ad 100 | ad 100 | ad 100 |

## Patentansprüche

1. Kosmetische Zubereitung zur dekorativen Anwendung auf der Haut, welche ein oder mehrere Farbpigmente enthält und **dadurch gekennzeichnet ist, daß** sie ferner ein oder mehrere Ligninsulfonate enthält.

2. Zubereitung nach Anspruch 1, **dadurch gekennzeichnet, daß** sie in Form von O/W-Emulsionen vorliegt.

3. Zubereitung nach einem der vorhergehenden Ansprüche, **dadurch gekennzeichnet, daß** als Ligninsulfonat Calcium- und/oder Natriumligninsulfonat und/oder Ammoniumligninsulfonat (CAS-Nr. 8061-53-8) gewählt wird.

4. Zubereitung nach einem der vorhergehenden Ansprüche, **dadurch gekennzeichnet, daß** der Gehalt an einem oder mehreren Ligninsulfonaten aus dem Bereich von 0,01 bis 20 Gew.-% - bezogen auf das Gesamtgewicht der Zubereitung - gewählt wird.

5. Zubereitung nach einem der vorhergehenden Ansprüche, **dadurch gekennzeichnet, daß** der Gehalt an einem oder mehreren Ligninsulfonaten aus dem Bereich von 0,5 bis 15 Gew.-% - bezogen auf das Gesamtgewicht der Zubereitung - gewählt wird.

6. Zubereitung nach einem der vorhergehenden Ansprüche, **dadurch gekennzeichnet, daß** der Gehalt an einem oder mehreren Ligninsulfonaten aus dem Bereich von 1 bis 7 Gew.-% - bezogen auf das Gesamtgewicht der Zubereitung - gewählt wird.

7. Zubereitung nach einem der vorhergehenden Ansprüche, **dadurch gekennzeichnet, daß** der Gehalt an einem oder mehreren Farbpigmenten aus dem Bereich von 0,01 bis 20 Gew.-% - bezogen auf das Gesamtgewicht der Zubereitung - gewählt wird.

8. Zubereitung nach einem der vorhergehenden Ansprüche, **dadurch gekennzeichnet, daß** der Gehalt an einem oder mehreren Farbpigmenten aus dem Bereich von 0,5 bis 15 Gew.-% - bezogen auf das Gesamtgewicht der Zubereitung - gewählt wird.

9. Zubereitung nach einem der vorhergehenden Ansprüche, **dadurch gekennzeichnet, daß** der Gehalt an einem oder mehreren Farbpigmenten aus dem Bereich von 1 bis 10 Gew.-% - bezogen auf das Gesamtgewicht der Zubereitung - gewählt wird.
